# EUROPEAN PATENT APPLICATION

(11) **EP 2 281 556 A1**
(43) Date of publication of application: **09.02.2011**
(21) Application number: 10179258.8
(22) Date of filing: 16.02.2006
(51) Int. Cl.: A61K 9/20

(54) **Tablets with improved drugs substance dispersibility**

(30) Priority: 25.02.2005 EP 05101458
(62) Divisional of application: 06706996.3
(71) Applicant: F. Hoffmann-La Roche AG, 4070 Basel (CH)
(72) Inventor: Bernigal, Nathalie, 68760, Willer sur Thur (FR); Garcia, Eric, 68700, Uffholtz (FR); Page, Susanne, 79540, Loerrach (DE); Tardio, Joseph, 68300, St. Louis (FR)
(74) Representative: Salud, Carlos E.

(57) **Abstract**

The present invention relates to a method for the preparation of pharmaceutical compositions in the form of tablets with improved drug substance dispersibility, said method being **characterized in that** it comprises the steps of: a)preparing a dispersion of at least one pharmaceutically active drug substance and at least one surfactant and/or binder in a liquid; b)preparing a carrier by dry blending one or more excipient(s) including at least one porous carrier; and c)spray granulating the dispersion prepared under a) onto the carrier prepared under b) in order to obtain a spray-granulated product.

## Description

The present invention relates to a method for the preparation of pharmaceutical compositions in the form of tablets with improved drug substance dispersibility, said method being **characterized in that** it comprises the steps of:
a) preparing a dispersion of at least one pharmaceutically active drug substance and at least one surfactant and/or binder in a liquid;
b) preparing a carrier by dry blending one or more excipient(s) including at least one porous carrier; and
c) spray granulating the dispersion prepared under a) onto the carrier prepared under b) in order to obtain a spray-granulated product.

The invention also encompasses tablets with improved drug substance dispersibility obtained by the method of the invention.

For low soluble drug substances in drug products with high drug loads it has been surprisingly found that the method of the invention results in tablets showing a good wettability, and an improved drug substance dispersibility which allow an immediate release of the drug substance and prevents sintering effects during compression.

In the field of pharmaceutical technology formulation the issues to be solved are mostly determined by the physico-chemical properties of the pure drug substance (wettability, solubility,...) or other important additives intended to be present in the end formulation. Many dosage forms are known to the pharmaceutical market, the most important being tablets and capsules. The formulation of a poor soluble drug substance especially in a formulation with high drug load is one of the major challenges in formulation development. The key parameters of success for such a formulation are:
- a good dispersibility,
- a good wettability of the tablet prior to tablet disintegration,
- a good wettability of the of the drug substance after tablet disintegration,
- and prevention of sintering due to compression.

As defined herein, the expression "drug substance having a low solubility" means drug substances having a low or poor solubility and classified according to the Biopharmaceutical Classification System (BCS) as class II or IV drug substances, as described in "Guidance for Industry, Waiver of In Vivo Bioavailability and Bioequivalence Studies for Immediate-Release Solid Oral Dosage Forms Based on a Biopharmaceutics Classification System", U.S. Department of Health and Human Services, Food and Drug Administration, Center for Drug Evaluation and Research (CDER), August 2000.

It is to be understood that the excipients used in the method according to the invention, including surfactants, wetting agents, binders, lubricants, disintegrating agents, carriers, fillers, etc. are of pharmaceutically acceptable grade.

The expression "a good dispersibility or micro-disintegration of the drug substance" means that the drug substance is released from the formulation in nearly the same particle size as it was used for manufacturing the formulation.

The expression "a good wettability" means that the solid/vapor interface is rapidly and completely replaced by a solid/liquid interface thus allowing a good dispersion of the particles in the surrounding liquid.

The expression "pharmaceutically active drug substance(s)" or "drug substance(s)" is indifferently used in the present patent application to denote a pharmaceutically active principle which is intended to cure and/or prevent illnesses. Any poor soluble drug substance can be formulated with the method of the invention; in particular drug substances selected from the group of BCS (Biopharmaceutical Classification System) class II/IV drug substances. The drug substance is generally present in the tablet with a weight percentage ranging from 25 to 70% of the weight of the tablet. In certain embodiments of the invention, the drug substance is a NK1 receptor antagonist or a MAOB inhibitor. NK1 receptor antagonists can be selected from those compounds and groups of compounds as specifically disclosed in EP 1035115, WO 00/50401, WO 00/50398, WO 00/53772, WO 00/73279, WO 00/73278, EP 1103546, EP 1103545, WO 01/90083, WO 01/94346, WO 02/06236, WO 02/08232, WO 02/16324, WO 02/47663, WO 02/42280, WO 02/079134, WO 02/062784, WO 02/092604, WO 02/0854458, WO 01/52844, WO 03/006016, WO 03/011860, WO 2004/067007, EP 0941092, EP 0941093, and EP 1082959 and especially in the claims thereof. MAOB inhibitors can be selected from those compounds and groups of compounds as specifically disclosed in WO 03/066596, WO 03/080573, WO 2004/014856, WO 03/091219, WO 2004/054985, WO 03/099763, WO 03/106380, WO 2004/007429, WO 2004/026826, WO 2004/026827 and WO 2004/026825 and especially in the claims thereof.

The expression "no sintering effect" means that there is no aggregation of the drug substance neither due to melting, partially melting nor due to plastic deformation.

The dispersion of step a) according to the method of the invention can be conducted using conventional methods and equipment. In certain embodiments of the invention, the dispersion is prepared under vacuum using a mixer-homogenizer apparatus equipped with a vacuum chamber such as e.g. a Moltomat MMV 20™. The resulting dispersion of step a) has preferably a dynamic viscosity of less than 150 mPa*s, preferably less than 100 mPa*s and still more preferably less than 75 mPa*s as measured with a conventional rheometer. A relative low viscosity allows a direct utilization of the dispersion after its preparation.

Any conventional pharmaceutically acceptable surfactant(s) and/or binder(s) may be used for preparing the dispersion of step a) according to the invention. The weight percentage of surfactant(s) present in the tablet generally ranges from 0 to 15 % of the total weight of the tablet whereas the weight percentage of binder(s) present in the tablet generally ranges from 4 to 15 % of the total weight of the tablet.

In a concern for clarity, it is hereby specified that the wording "surfactant(s) and/or binder(s)" in step a) means that the dispersion of step a) may comprise:
- either one or more surfactant(s),
- or one or more binder(s),
- or a mixture of one or more surfactant(s) and of one or more binder(s).

Non-limiting examples of surfactants include anionic surfactants, co-emulsifiers, cationic surfactants, non-ionic surfactants, and amphoteric surfactants. Further examples include sodium lauryl sulfate, docusate sodium, caseinate sodium, salts of fatty acids, quaternary amines, cetylpyridiniumchloride, polyoxyethylene fatty acid esters, cetyl alcohol, fatty acid esters, cetostearyl alcohol, cholesterol, sorbitan fatty acid esters, polysorbats, poloxamers, phospholipids and preferably sucrose fatty acid esters and tocopheryl polyethylene glycol succinate.

Non-limiting examples of binders include cellulose, derivates and salts thereof such as carboxymethylcellulose sodium, ethylcellulose, hydroxypropyl methylcellulose, methylcellulose, hydroxy ethylcellulose, hydroxypropyl cellulose, and microcrystalline cellulose, or starch and modified starch, solid or liquid glucose, gelatin, and preferably polyvinylpyrrolidone (PVP), or PVP/VA copolymer.

The dispersion of step a) can also comprise a mixture of one or more of the hereinabove recited surfactant(s) and binder(s).

Further other conventional pharmaceutically acceptable excipients may be added in step a). Non-limiting examples of such excipients include conventional pharmaceutically acceptable wetting agents and solubilizers.

As for the liquid used in step a) any pharmaceutically acceptable liquid may be used including water or a mixture of water and an alcohol such as ethanol in *quantum satis.* Mixtures of water and an alcohol are mixtures of 0 to 100 weight percent of water and 100 to 0 weight percent of alcohol, for example 0 to 75 weight percent of water and 100 to 25 weight percent of alcohol.

The process of the invention also encompasses a process comprising the steps of:
a) preparing a homogenous dispersion of at least one pharmaceutically active drug substance and at least one surfactant and/or binder in a liquid;
b) preparing a carrier by dry blending one or more excipient(s) including at least one porous carrier; and
c) spray granulating the homogenous dispersion prepared under a) onto the carrier prepared under b) in order to obtain a spray-granulated product.

In certain embodiments of the invention, step a) is split in two sub-steps a1) and a2) which may be conducted as follows:
a1) dispersing the either the binder(s) or the surfactant(s) or mixture thereof in the liquid;
a2) wetting and dispersing the drug substance(s) with the dispersion of step a1) and optionally add further excipients.

Dry blending in step b) can be performed using any conventional methods and equipment, for example using a conventional tumble mixer, subsequently sieving the resulting mixture and then mixing again with the tumble mixer.

As already mentioned hereinabove the preparation of a carrier by dry blending in step b) involves one or more excipient(s) including at least one porous carrier. The total weight percentage of the carrier, including the porous carrier used in step b) generally ranges from 20 to 65% of the weight of the tablet.

Any suitable porous carrier may be used and some of these suitable porous carriers are directly commercially available, such as colloidal silicon dioxide, for example sold under the trademark Aerosil™. The weight percentage of the porous carrier generally ranges from 0.5 to 10% of the weight of the tablet.

It is understood that, in addition to the porous carrier, other excipients may compose the rest of the carrier. Such excipients are e.g. fillers and/or disintegrating agents.

Suitable fillers or diluents for preparing the carrier of step b) include but are not limited to calcium phosphates, calcium sulfates, carboxymethylcellulose calcium, cellulose, cellulose acetate, dextrates, dextrin, dextrose, e.g. glucose, ethylcellulose, fructose, glyceryl palmitostearate, hydrogenated vegetable oil, kaolin, lactitol, lactose, e.g. lactose monohydrate, magnesium carbonate, magnesium oxide, maltitol, maltodextrin, maltose, microcrystalline cellulose, polymethacrylates, powdered cellulose, pregelatinized starch, silicified microcrystalline cellulose, sodium chloride, sorbitol, starch and modified starch, sucrose, sugar and talc.

Suitable disintegrating agents for preparing the carrier of step b) include but are not limited to alginic acid, carboxymethylcellulose, cellulose, magnesium aluminium silicate, methylcellulose, microcrystalline cellulose, potassium, polacrilin, povidone, sodium alginate, sodium starch glycolate, starch and preferably colloidal silicon dioxide, croscarmellose sodium or crospovidone.

Spray granulation of step c) according to the method of the invention can be performed using conventional methods and equipment as well. In certain embodiments of the invention, spray granulation is performed in a fluid bed granulator such as e.g. of the type Aeromatic Fielder T/SG2.

The spray-granulated product of step c) can then be compressed in tablets and then film-coated with the following steps of:
d) dry mixing the spray-granulated product of c) with one or more excipient(s), said excipient(s) forming an external phase, in order to obtain a final blend;
e) compressing the final blend of step d) to tablets;
f) film-coating the tablets of step e).

Compression of the final blend to tablets can be performed using conventional methods and equipment. In certain embodiment of the invention, the compression is performed using a Korsch PH 250 and a conventional rotary feeder.

Any pharmaceutically acceptable excipient can be used in the final blend of step d). Examples of such excipients include but are not limited to glidants and lubricants as well as further excipients such as excipients improving the compression behavior (e.g. mannitol, silicified microcrystalline cellulose). The total weight percentage of the glidants, lubricants and other excipients used for the tablet generally ranges from 0.5 to 10% of the weight of the tablet.

Any pharmaceutically acceptable excipient can be used for the external phase of the tablet, preferably conventional glidants and lubricants. Suitable glidants can be selected from the group consisting of magnesium trisilicate, powdered cellulose, starch, tribasic calcium phosphate and preferably talc. Suitable lubricants can be selected from the group consisting of calcium stearate, canola oil, glyceryl palmitostearate, hydrogenated vegetable oil, magnesium oxide, mineral oil, poloxamer, polyethylene glycol, polyvinyl alcohol, sodium benzoate, sodium lauryl sulfate, stearic acid, zinc stearate and preferably talc, sodium stearyl fumarate or magnesium stearate.

As already mentioned above, once compressed, the tablets with improved drug substance dispersibility of the invention can be film-coated. The film-coating mainly comprises polymers as well as further other excipients such as plastizicer, coloring agents, talc and additional excipients.

Film coating can be performed using any conventional method and equipment, for example using a Glatt GC550™ apparatus equipped with a Watson Marlow™ pump.

The invention also encompasses tablets with improved drug substance dispersibility obtained by the method of the invention. The tablets with improved drug substance dispersibility obtained by the method of the invention are best defined by their method of preparation that is to say the method of the invention.

For low soluble drug substances in drug products with high drug loads it has been surprisingly found that the method of the invention results in tablets showing a good wettability, and an improved dispersibility of the drug substance which allow an immediate release of the drug substance and prevents sintering effects during compression.

These surprising improved results were achieved because of the originality of steps a) to c) of the method according to the invention.

Conventionally, the methods for the preparation of tablets of the prior art do not follow the original steps a) to c) of the method of the invention.

Without being bound by any theory, the Applicant believes that the good dispersibility and wettability could be achieved by dispersing the drug substance together with the surfactant(s) and/or the binder(s) in the liquid substance. In addition, it is believed that the good dispersibility and fast disintegration of the tablets according to the invention results from the addition of a porous carrier in the dry blend of the carrier.

The tablets with improved drug substance dispersibility obtainable, preferably obtained by the process of the invention are mainly characterized by:
- a disintegration time in water of less than 20 minutes, preferably less than 15 minutes, more preferably of less than 10 minutes as measured in a conventional disintegration test as described in the Pharmacopoeia;
- a high initial dissolution rate or kinetic after pre-incubation under non-sink conditions as in-vitro parameter, characterizing the dispersibility. The definition of the dispersibility behavior of different formulations can be measured by the determination of the initial dissolution kinetic, after pre-incubation. A high dipsersiblity (single crystals) leads to fast initial dissolution rate. With this procedure sintering effects (particle agglomeration, particle size increase) can be indentified by comparison of the initial dissolution rate of the granulate (before compression) and the tablets (after tablet compression).
- For the measurement of the initial dissolution kinetics after preincubation under non-sink conditions the following standard test can be applied:
   Procedure:
      1. gastric stage: disintegration in a coarse suspension
         5 ml FeSGF (=fed state simulated gastric fluid) pH 5 (with 3 mg/ml SE-L1695= sucrose laurate Ryoto (L1695)) are added to a tablet portion (∼25mg API) in a 20 ml vial. The vial is incubated in a rotating shaker with 2 rpm at 37°C (mild mixing with low shear forces) during at least 30 min or up to 60 min until full disintegration is observed. Two suspension samples are taken and analyzed by HPLC as "100%" control.
   2. duodenal stage: dissolution kinetics from the dispersed API
      a sample from (1.) is diluted in the same medium or in FeSSIF pH5 (=fed state simulated small intestine fluid) and stirred at ∼50rpm. The drug product concentration for the measurements has to be adapted, based on the drug substance characteristics (eg. for example 1: The solubility in the FeSGF medium is 32 µg/ml. The sample contains ∼250 µg/ml API (dilution rate of 1/20)). Samples are taken at different time points (i.e. 1,3,5 and 8 min) and immediately filtered with a 0.45µm Millex-HV4 filter and analyzed by HPLC.

The following examples are intended to further illustrate the composition and method of the invention without restricting them to the embodiments exemplified.

Examples of tablets with improved drug substance dispersibility and methods according to the invention

### Example 1

Table I hereafter exhibits a composition for tablets with improved drug substance dispersibility according to the invention:

**Table I**

| Step | Ingredient | Function | Amount (%) | Amount (mg) |
|---|---|---|---|---|
| Step a) | NK1 receptor antagonist (R673) | Drug substance | 52.63 | 400.00 |
| Step b) | Sucrose fatty acid ester (Sucrosemonopalmitate P 1670) | Surfactant | 5.26 | 40.00 |
| | PVP/VA copolymer (Plasdone S 630) | Binder | 9.21 | 70.00 |
| | Lactose monohydrate | Fillers | 1.11 | 8.45 |
| | Pregelatinized starch (STARX 1500) | | 13.16 | 100.00 |
| | Colloidal silicon dioxide (Aerosil 200) | Porous carrier | 6.58 | 50.00 |
| | Croscarmellose Sodium (AC DI SOL) | Disintegrating agent | 3.95 | 30.00 |
| External phase of the tablet | Mannitol (Parteck M 200) | Filler | 3.29 | 25.00 |
| | Magnesium Stearate | Lubricants | 0.64 | 4.85 |
| | Sodium stearyl fumarate | | 1.28 | 9.70 |
| | Talc | | 2.89 | 22.00 |
| Total | | | 100.00 | 760.00 |

The disintegration time of the composition of table I were assayed as described hereinabove.

The disintegration time in water as well as in 0.1 N HCl was less than 10 min.

The initial dissolution rate after 1 min was greater than 25% of saturation and after 3 min greater than 45% of saturation.

The tablet with improved drug substance dispersibility of table I was prepared according to the following method of the invention:
Step a) Preparing an aqueous dispersion of PVP/VA 64 copolymer, the Sucrose fatty acid ester and R673 under vacuum using the Moltomat MMV 20.
Step b) Blending of lactose monohydrate, pregelatinized starch, colloidal silicon dioxide (porous carrier) and Croscarmellose Sodium using a tumble mixer for 5 min.
Step c) Spray granulation of the dispersion prepared under a) onto the the dry powder mix prepared under b) using a fluid bed granulator (type WST SG2) as top spray process. For the spray granulation, the following parameters were used:
   - inlet air temperature of about 65 to 70°C,
   - air flow rate of about 150 to 200 m³/h,
   - spray rate of about 100 to 250 g/min,
   - atomizing air pressure of about 2.5 bar.
Step d) The dry sieved material was mixed with Mannitol in a tumble mixer for 10 min. Then the other excipients (Magnesium Stearate, Sodium stearyl fumarate and Talc) were mixed with a part of the material using a tumble mixer for 3 min. Afterwards the remaining parts of the material was added and blended for 5 min using a tumble mixer.
Step e) The final blend prepared under d) was compressed into tablets of oval shape (18 mm x 8.33 mm) using a Korsch PH 250 (60rpm, 12-13 kN).
Step f) The tablets prepared under e) were film-coated using a commercially available film-coating system. The coating step was performed using Glatt GC 550.

### Example 2

Table II hereafter exhibits another tablet with improved drug substance dispersibility according to the invention:

| Step | Ingredient | Function | Amount (%) | Amount (mg) |
|---|---|---|---|---|
| Step a) | MAOB inhibitor (R1500) | Drug substance | 25.90 | 51.81 |
| | PVP/VA copolymer (Plasdone S 630) | Binder | 9.21 | 18.42 |
| | Sucrose fatty acid ester (Sucrosemonopalmitate P 1670) | Surfactant | 5.26 | 10.52 |
| Step b) | Lactose monohydrate | Fillers | 27.33 | 54.65 |
| | Pregelatinized starch (STARX 1500) | | 13.60 | 27.20 |
| | Colloidal silicon dioxide (Aerosil 200) | Porous carrier | 3.95 | 7.90 |
| | Croscarmellose Sodium (AC DI SOL) | Disintegrating agent | 6.60 | 13.20 |
| External phase of the tablet | Mannitol (Parteck M 200) | Filler | 3.30 | 6.60 |
| | Magnesium Stearate | Lubricants | 0.65 | 1.30 |
| | Sodium stearyl fumarate | | 1.30 | 2.60 |
| | Talc | | 2.90 | 5.80 |
| Total | | | 100.00 | 200.00 |

The disintegration time of the composition of table II were assayed as described hereinabove.

The disintegration time in water was less then 7 min.

The tablet with improved drug substance dispersibility_of table II was prepared according to the following method of the invention:
Step a) Preparing an aqueous dispersion of PVP/VA 64 copolymer, the Sucrose fatty acid ester and R1500 using a Polytron.
Step b) Blending of lactose monohydrate, pregelatinized starch, colloidal silicon dioxide (porous carrier) and Croscarmellose Sodium using a tumble mixer.
Step c) Spray granulation of the dispersion prepared under a) onto the the dry powder mix prepared under b) using a fluid bed granulator (type Strea-1) as top spray process.
Step d) The dry sieved material was mixed with Mannitol. Then the other excipients (Magnesium Stearate, Sodium stearyl fumarate and Talc) were mixed with a part of the material using a tumble mixer for 3 min. Afterwards the remaining parts of the material was added and blended for 3 min using a tumble mixer.
Step e) The final blend prepared under d) was compressed into tablets of oval shape (11.5 mm x 6 mm) using a Korsch PH 250 (60rpm, 9 kN).
Step f) no film-coating was applied.

### Example 3

Table III hereafter exhibits still another tablets with improved drug substance dispersibility according to the invention:

| Step | Ingredient | Function | Amount (%) | Amount (mg) |
|---|---|---|---|---|
| Step a) | MAOB inhibitor (R1500) | Drug substance | 25.77 | 51.54 |
| | PVP/V A copolymer (Plasdone S 630) | Binder | 10.00 | 20.00 |
| | Sucrose fatty acid ester (Sucrosemonopalmitate P 1670) | Surfactant | 6.25 | 12.50 |
| Step b) | Lactose monohydrate | Fillers | 28.98 | 57.96 |
| | Microcrystalline cellulose (Avicel PH 102) | | 15.00 | 30.00 |
| | Colloidal silicon dioxide (Aerosil 200) | Porous carrier | 7.00 | 14.00 |
| | Crospovidone | Disintegrating agent | 5.00 | 10.00 |
| External phase of the tablet | Magnesium Stearate | Lubricants | 0.50 | 1.00 |
| | Talc | | 1.50 | 3.00 |
| Total | | | 100.00 | 200.00 |

The disintegration time of the composition of table III were assayed as described hereinabove.

The disintegration time in water was less than 15 min.

The tablet with improved drug substance dispersibility of table III was prepared according to the following method of the invention:
Step a) Preparing an aqueous dispersion of PVP/VA 64 copolymer, the Sucrose fatty acid ester and R1500 using a Polytron.
Step b) Blending of lactose monohydrate, microcrystalline cellulose, colloidal silicon dioxide (porous carrier) and Crospovidone using a tumble mixer.
Step c) Spray granulation of the dispersion prepared under a) onto the the dry powder mix prepared under b) using a fluid bed granulator (type Strea-1) as top spray process.
Step d) A part of the dry sieved material was mixed with Magnesium Stearate and Talc using a tumble mixer for 3 min. Afterwards the remaining part of the granules was added and blended for 3 min using a tumble mixer.
Step e) The final blend prepared under d) was compressed into tablets of oval shape (11.5 mm x 6 mm) using a Korsch PH 250 (60rpm, 8 kN).
Step f) no film-coating was applied.

## Claims

1. A method for the preparation of pharmaceutical compositions in the form of tablets with improved drug substance dispersibility, said method being **characterized in that** it comprises the steps of:
a) preparing a dispersion of at least one pharmaceutically drug substance and at least one surfactant and/or binder in a liquid;
b) preparing a carrier by dry blending one or more excipient(s) including at least one porous carrier; and
c) spray granulating the dispersion prepared under a) onto the carrier prepared under b) in order to obtain a spray-granulated product.

2. The method according to claim 1, further comprising the steps of:
d) dry mixing the spray-granulated product of c) with one or more excipient(s), said excipient(s) forming an external phase, in order to obtain a final blend;
e) compressing the final blend of step d) to tablets;
f) film-coating the tablets of step e).

3. The method according to any one of claim 1 or 2, wherein in step a), the dispersion comprises at least one surfactant.

4. The method according to claim 3, wherein the surfactant(s) is (are) selected from the group consisting of non-ionic surfactants, and amphoteric surfactants, comprising sodium lauryl sulfate, docusate sodium, caseinate sodium, salts of fatty acids, quaternary amines, cetylpyridiniumchloride, polyoxyethylene fatty acid esters, cetyl alcohol, fatty acid esters, cetostearyl alcohol, cholesterol, sorbitan fatty acid esters, polysorbats, poloxamers, phospholipids and preferably sucrose fatty acid esters and tocopheryl polyethylene glycol succinate.

5. The method according to any one of claim 1 or 2, wherein in step a), the dispersion comprises at least one binder.

6. The method according to claim 5, wherein the binder(s) is (are) selected from the group consisting of cellulose, derivates and salts thereof such as carboxymethylcellulose sodium, ethylcellulose, hydroxypropyl methylcellulose, methylcellulose, hydroxy
ethylcellulose, hydroxypropyl cellulose, and microcrystalline cellulose, or starch and modified starch, solid or liquid glucose, gelatin, and preferably polyvinylpyrrolidone (PVP), or a PVP/VA copolymer.

7. The method according to any one of claim 1 or 2, wherein in step a), the dispersion comprises a mixture of at least one surfactant and at least one binder.

8. The method according to claim 7, wherein the mixture comprises sucrose fatty acid ester as a surfactant and a PVP/VA copolymer as a binder.

9. The method according to any one of claims 1 to 8, wherein in step b) the dry bled comprises fillers and/or disintegrating agents and a porous carrier.

10. The method according to any one of claims 9, wherein the porous carrier is colloidal silicon dioxide.

11. The method according to claim 9 or 10, wherein the filler is selected from the group consisting of calcium phosphates, calcium sulfates, carboxymethylcellulose calcium, cellulose, cellulose acetate, dextrates, dextrin, dextrose, glucose, ethylcellulose, fructose, glyceryl palmitostearate, hydrogenated vegetable oil, kaolin, lactitol, lactose, lactose monohydrate, magnesium carbonate, magnesium oxide, maltitol, maltodextrin, maltose, microcrystalline cellulose, polymethacrylates, powdered cellulose, pregelatinized starch, silicified microcrystalline cellulose, sodium chloride, sorbitol, starch and modified starch, sucrose, sugar and talc.

12. The method according to claim 9 or 10, wherein the disintegrating agent is selected from the group consisting of alginic acid, carboxymethylcellulose, cellulose, magnesium aluminium silicate, methylcellulose, microcrystalline cellulose, potassium, polacrilin, povidone, sodium alginate, sodium starch glycolate, starch and preferably colloidal silicon dioxide, croscarmellose sodium or crospovidone.

13. The method according to any one of claims 1 to 12, wherein in step a), the dispersion for the spray granulation comprises the steps of:
a1) dispersing either the binder(s) or the surfactant(s) or mixture thereof in the liquid;
a2) wetting and dispersing the drug substance(s) with the dispersion of step a1) and optionally add further excipients.

14. The method according to any one of claims 1 to 13, wherein the preparation of the dispersion of step a) is conducted under vacuum.

15. The method according to claim 14, wherein the resulting dispersion of step a) has a viscosity of less than 150 m Pa/s, preferably less than 100 m Pa/s and still more preferably less than 75 m Pa/s.

16. A tablet with improved drug substance dispersibility obtained by a method according to any one of claims 1 to 15.

17. The tablet with improved drug substance dispersibility according to claim 16 having a disintegration time in water of less than 20 minutes, preferably less than 15 minutes, more preferably of less than 10 minutes and a high initial dissolution rate.

18. The invention as described hereinabove.
